# EUROPEAN PATENT APPLICATION

(11) **EP 4 725 587 A1**
(43) Date of publication of application: **15.04.2026**
(21) Application number: 25187536.5
(22) Date of filing: 04.07.2025
(51) Int. Cl.: B01D 53/14

(54) **ACID GAS ABSORBENT, METHOD FOR REMOVING ACID GAS, AND ACID GAS REMOVAL APPARATUS**

(30) Priority: 10.10.2024 JP 2024177832
(71) Applicant: Kabushiki Kaisha Toshiba, Tokyo 105-0023 (JP); Toshiba Energy Systems & Solutions Corporation, Saiwai-ku Kawasaki-shi Kanagawa (JP)
(72) Inventor: NAKANO, Yoshihiko, Minato-ku, Tokyo, 105-0023 (JP); SAITO, Hitomi, Minato-ku, Tokyo, 105-0023 (JP); SUZUKI, Akiko, Minato-ku, Tokyo, 105-0023 (JP); YOSHIMURA, Reiko, Minato-ku, Tokyo, 105-0023 (JP); MURAI, Shinji, Kawasaki-shi, Kanagawa, 212-0013 (JP); FUJITA, Kento, Kawasaki-shi, Kanagawa, 212-0013 (JP)
(74) Representative: Hoffmann Eitle

(57) **Abstract**

According to an embodiment, an acid gas absorbent includes a solvent, and at least one or more amine compounds selected from the group consisting of amine compounds represented by chemical formula (1a).

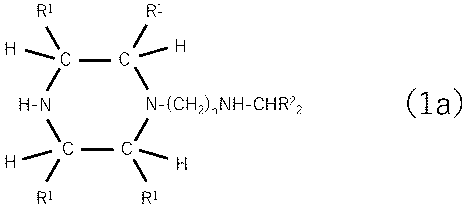

Wherein R¹'s are each independently hydrogen or an unsubstituted alkyl group having 3 or less carbon atoms, R²' s are each independently an unsubstituted alkyl group having 3 or less carbon atoms, n is either 2 or 3.

## Description

### CROSS-REFERENCE TO RELATED APPLICATION(S)

This application is based upon and claims the benefit of priority from Japanese Patent Application No. 2024-177832, filed October 10, 2024, the entire contents of which are incorporated herein by reference.

### FIELD

Embodiments described herein relate generally to an acid gas absorbent, a method for removing an acid gas, and an acid gas removal apparatus.

### BACKGROUND

In recent years, a greenhouse effect caused by an increase in carbon dioxide (CO₂) concentration has been pointed out as one factor for global warming phenomena, and international measures for protecting the environment on a global scale are urgently needed. CO₂ is generated mainly by industrial activities, and there is a growing momentum to suppress emission of CO₂ to the environment.

As a technique for suppressing an increase in concentration of an acid gas such as CO₂, there are indicated development of an energy saving product, use of an acid gas as a resource, a technique of isolating and storing an acid gas, and conversion to alternative energy, such as natural energy or nuclear energy, which does not discharge an acid gas. As one of these techniques, a technique of separating and recovering a discharged acid gas is known.

Acid gas separation techniques that have been studied so far include absorption methods, adsorption methods, membrane separation methods, and deep cooling methods. Among them, the absorption method is suitable and economical for efficiently treating a large amount of gas, and it is easy to increase a size of a removal apparatus. Therefore, application of the absorption method to factories and power plants has been studied.

As a method intended for a thermal power plant or the like using fossil fuel, mainly, a method of removing and recovering CO₂ in a combustion exhaust gas by bringing exhaust gas generated when burning fossil fuel (coal, oil, natural gas, etc.) into contact with a chemical absorbent, and, further, a method for storing recovered CO₂ are known. In addition, it has been proposed to remove an acid gas such as hydrogen sulfide (H₂S) in addition to CO₂ using a chemical absorbent.

In general, alkanolamines typified by monoethanolamine (MEA) are known as chemical absorbents used in the absorption method. Such alkanolamines have been developed since the 1930s and are still used today. Common alkanolamines used in the absorption method include 2-amino-2-methylpropanolamine, methylaminoethanol, ethylaminoethanol, propylaminoethanol, diethanolamine, methyldiethanolamine, dimethylethanolamine, diethylethanolamine, triethanolamine, and dimethylamino-1-methylethanol.

When these conventionally used alkanolamines are used alone, a CO₂ absorption rate may not be sufficient, and, usually, a compound having a reaction promoting effect is often used in combination. As such a compound having a reaction promoting effect, a cyclic diamine is known, but which generally has a high vapor pressure, is easily dissipated, and may have poor handleability in some cases. In addition, for recovery of carbon dioxide, a step of absorbing carbon dioxide into an aqueous amine solution and a step of releasing carbon dioxide from an aqueous solution that has absorbed carbon dioxide are performed with high efficiency. Also, recovery energy consumed for recovery of carbon dioxide during the steps is required to be low. In order to meet this requirement, an absorbent having a large amount of carbon dioxide to be absorbed is effectively used. At the same time, it is required, from the viewpoint of influences on the environment to suppress diffusion of an amine to be released to the atmosphere as much as possible. For example, JP 2006-518662 T discloses an absorbent for removing an acid gas from a fluid. The absorbent contains a combination of a tertiary alkanolamine and hydroxyethyl piperazine or the like.

On the other hand, in the CO₂ recovery system described above, the exhaust gas (acid gas) contains oxygen, and, for the CO₂ release (recovery) from the absorbent that has absorbed the acid gas, the absorbent is often heated at 100°C or higher. Under such an environment, it is known that the amine in the absorbent is oxidized by oxygen, and deteriorates as the heating temperature increases.

For example, oxidation of the alkanolamine in the acid gas absorption step and an increase in deterioration due to an increase in heating temperature have been reported.

As described above, conventional absorbents have low durability (oxidation resistance), and thus have a problem that absorption characteristics deteriorate with time. Therefore, a new absorbent that simultaneously satisfies durability and an amount of an acid gas to be absorbed is required, as an acid gas absorbent.

### BREIF DESCRIPTION OF THE DRAWINGS

FIG. 1 is a schematic view of an acid gas removal apparatus according to an embodiment.
FIG. 2 is a graph showing adsorption performance and release performance of the acid gas absorbent according to the embodiment.

### DETAILED DESCRIPTION

In general, according to an embodiment, an acid gas absorbent contains: a solvent,
and at least one or more amine compounds selected from the group consisting of amine compounds represented by chemical formula (1a). 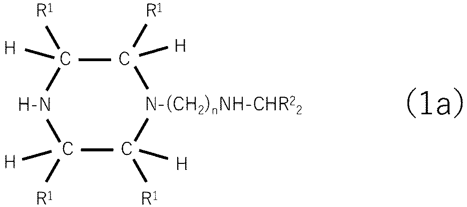 wherein:
R¹'s are each independently hydrogen or an unsubstituted alkyl group having 3 or less carbon atoms,
R²'s are each independently an unsubstituted alkyl group having 3 or less carbon atoms, and
n is either 2 or 3.

A method for removing an acid gas according to an embodiment includes bringing a gas containing an acid gas into contact with the acid gas absorbent to remove the acid gas from the gas containing the acid gas.

An acid gas removal apparatus according to an embodiment includes:
an absorber that removes an acid gas from a gas containing the acid gas by causing the acid gas absorbent to absorb the acid gas by contact between the gas containing the acid gas and the acid gas absorbent; and
a regenerator that releases the acid gas from the acid gas absorbent that has absorbed the acid gas to regenerate the acid gas absorbent,
in which the acid gas absorbent that has been regenerated by the regenerator is reused by the absorber.

Hereinafter, embodiments of the present invention will be described in detail. In the following embodiments, a case where the acid gas is carbon dioxide will be described as an example. However, the acid gas absorbent according to an embodiment of the present invention can provide the same effect also on other acid gases such as hydrogen sulfide. The acid gas absorbent according to the embodiment is particularly suitable for absorption of oxidized gases such as carbon dioxide and hydrogen sulfide.

The cyclic diamine compound which can be used in the acid gas absorbent according to the embodiment is represented by chemical formula (1a). 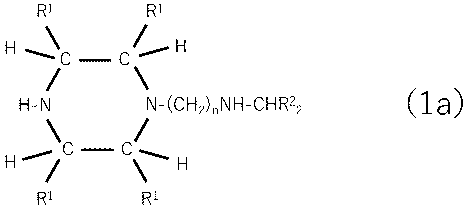 wherein:
R¹'s are each independently hydrogen or an unsubstituted alkyl group having 3 or less carbon atoms,
R²'s are each independently an unsubstituted alkyl group having 3 or less carbon atoms, and
n is either 2 or 3.

Specific examples of the amine compound represented by the chemical formula (1a) include the following compounds.

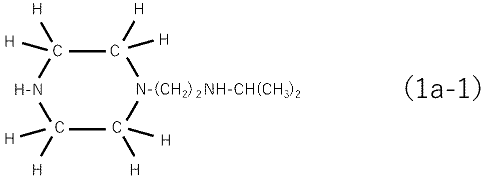

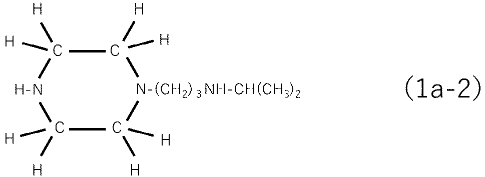

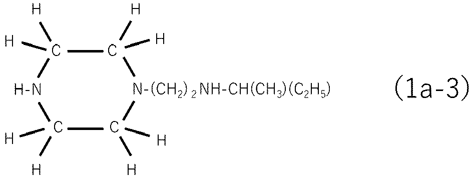

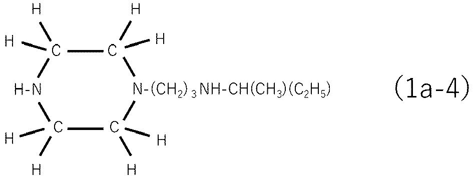

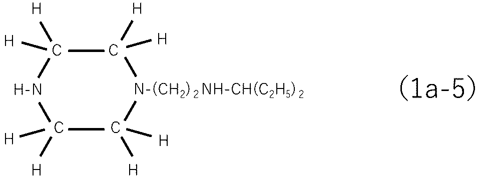

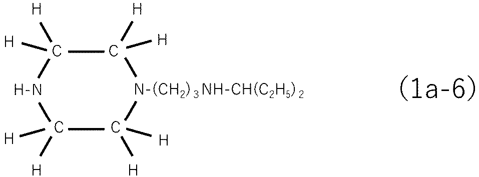

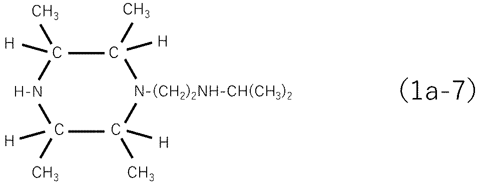

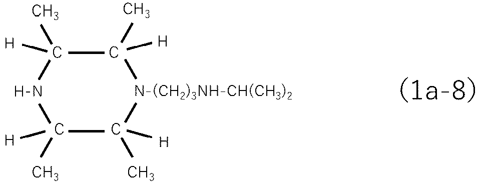

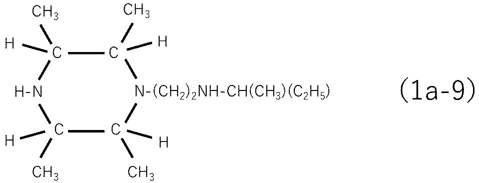

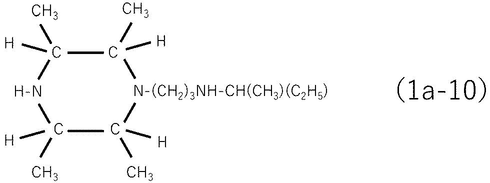

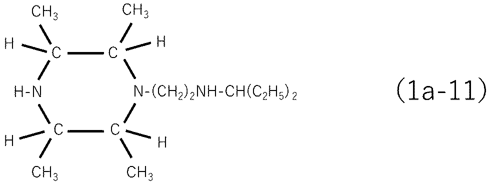

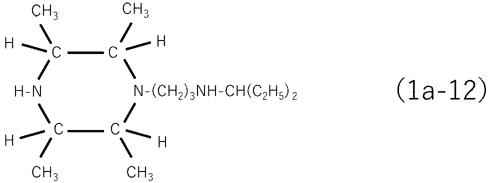

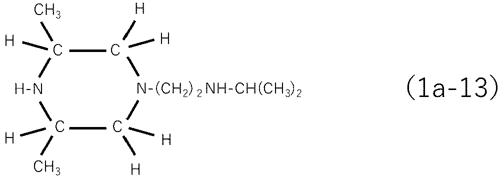

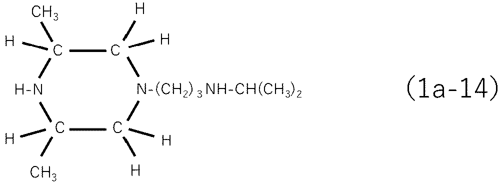

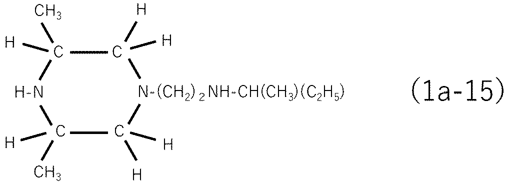

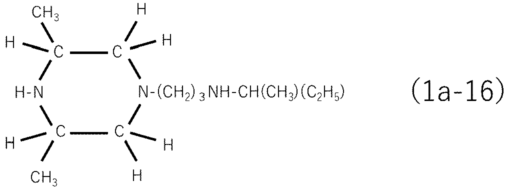

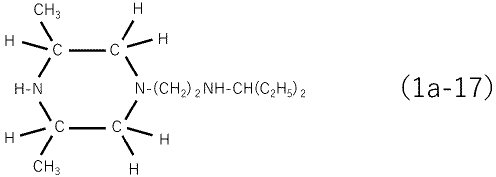

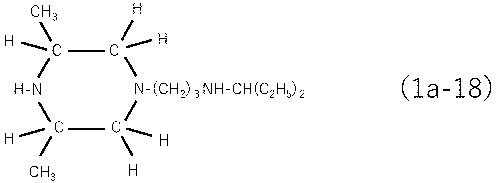

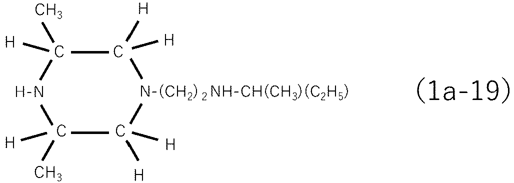

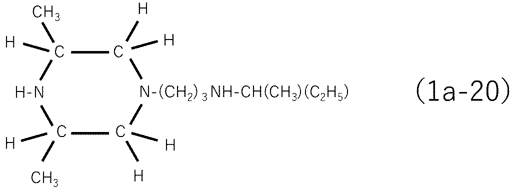

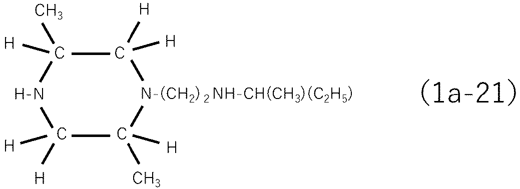

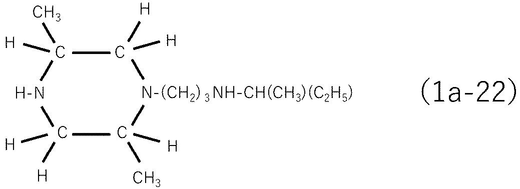

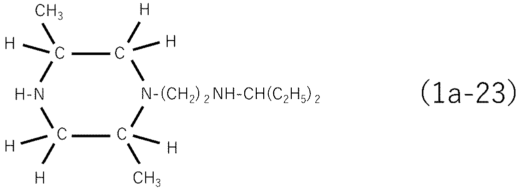

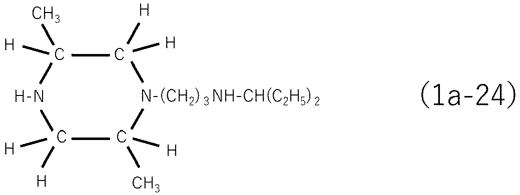

The acid gas absorbent according to the embodiment includes a chemical formula (1a). Here, two or more of the compounds represented by chemical formula (1a) may be combined. The method for producing the compound of the chemical formula (1a) will be described later.

In the acid gas absorbent according to the embodiment, in chemical formula (1a), all of R²'s are preferably unsubstituted alkyl groups having a carbon number of 1. This is because when the molecular weight of the whole of the chemical formula (1a) is small, the absorption amount per unit mass is improved, and both the dissolution and the oxidation resistance can be achieved. In this case, (CHR²₂) in the chemical formula (1a) is preferably an isopropyl group. In this case, both solubility and oxidation resistance can be further satisfied.

In the acid gas absorbent according to the embodiment, it is preferable that all of R¹'s in chemical formula (1a) are hydrogens. In this case, when all of R¹'s are hydrogens, the molecular weight of the whole of the chemical formula (1a) becomes smaller as compared with the case where alkyl groups are contained in R¹, and the absorption amount per unit mass is improved, and both the solubility and the oxidation resistance can be satisfied. In addition, n is 2 to 3, and n = 2 is preferable.

A total content rate of the cyclic diamine compounds represented by chemical formula (1a) contained in the acid gas absorbent according to the embodiment is preferably 10 mass% or more and 60 mass% or less, more preferably 20 mass% or more and 50 mass% or less, and still more preferably 30 mass% or more and 40 mass% or less based on the total weight of the acid gas absorbent. In general, the higher the content rate of the amine compound is, the larger amounts of carbon dioxide to be absorbed and to be released per unit volume are, and the higher carbon dioxide absorption rate and release rate are, which is thus preferable in terms of energy consumption and treatment efficiency. In general, when the content rate of the amine compound in the acid gas absorbent is too high, a viscosity of the absorbent is likely to increase. However, in the embodiment, when the content rate of the amine compound of chemical formula (1a) is 60 mass% or less, such a tendency is not observed. The content rate of the amine compound represented by chemical formula (1a) is set to 10 mass% or more, leading to attainment of a sufficient amount of carbon dioxide to be absorbed, a sufficient carbon dioxide absorption rate, and excellent treatment efficiency. When the acid gas absorbent in which the content rate of the amine compound represented by chemical formula (1a) is in the above range is used for carbon dioxide recovery, the acid gas absorbent is advantageous in long-term operation because of not only its large amount of carbon dioxide to be absorbed but also its high oxidation durability.

The acid gas absorbent according to the embodiment contains a solvent, and the amine compound is dissolved or dispersed therein. As the solvent, water, an organic solvent, or a mixed solvent thereof, for example, an aqueous solvent can be used. From the viewpoint of safety and cost, it is preferable to use water or an aqueous solvent as the solvent. However, in order to improve solubility of the amine compound and the like, an organic solvent or a mixed solvent having a relatively high organic solvent content can also be used. The aqueous solvent mainly contains water and contains a small amount of an organic solvent. However, when a boiling point of the organic solvent is low, the organic solvent may volatilize in an acid gas absorbing device to cause damage to the device. Therefore, the organic solvent has a boiling point of water, that is, 100°C or higher. When water is used as the solvent, a content rate thereof is preferably 40 mass% or more and 90 mass% or less, and particularly preferably 50 mass% or more and 80 mass% or less, based on the total mass of the acid gas absorbent. The content rate of water within this range is preferable from the viewpoint of suppressing an increase in viscosity of the absorbent and suppressing foaming when absorbing carbon dioxide. In addition, the aqueous solvent contains a small amount of organic solvent, but a content rate thereof is preferably 1 mass% or less based on the acid gas absorbent.

The acid gas absorbent according to the embodiment may further include at least one amine compound selected from the group consisting of amine compounds represented by the following chemical formula (1b), chemical formula (1c), and chemical formula (1d), in addition to the cyclic diamine compound. 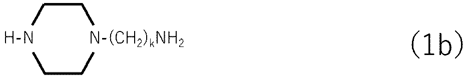 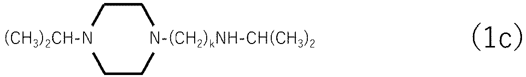 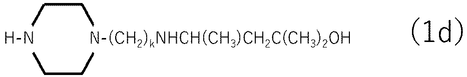 [In the chemical formula, k is each independently 2 to 3] In the chemical formula, k is each independently 2 to 3, and k = 2 is preferable.

In view of the balance between the amount of the acid gas absorbed and released and the oxidation resistance, the content of the amine compound represented by the chemical formula (1a) is preferably 10 mass% or more and 60 mass% or less, more preferably 20 mass% or more and 50 mass% or less, and still more preferably 30 mass% or more and 40 mass% or less based on the total amount of the acid gas absorbent. The content of the amine compounds represented by the chemical formulae (1b) to (1d) is preferably 1 mass% or more and 20 mass% or less, more preferably 1 mass% or more and 15 mass% or less, still more preferably 1 mass% or more and 10 mass% or less, and further preferably 3 mass% or more and 10 mass% or less. In addition, when the amount of the compounds of (1b) to (1d) is increased to 5 mass% to 15 mass%, an acid gas absorbent having the effects specific to the compounds of (1b) to (1d) can be obtained. For example, the absorption performance can be further improved by adding a large amount of the compound (1b). From such a viewpoint, the content is more preferably 10 mass% or more and 15 mass% or less.

The acid gas absorbent releases acid gas in an amount of 15NL or more and 40NL or less per unit amount (kg) at 70°C and 1atm for 40 minutes from the start of the release. The release is preferably 15NL or more and 35NL or less, more preferably 20NL or more and 35NL or less, and still more preferably 20NL or more and 30NL or less. When the amount is 20NL or more and less than 25NL, the acid gas absorbent exhibits a higher absorption performance. On the other hand, when the amount is 25NL or more and 30NL or less, the acid gas absorbent exhibits a higher release performance. The acid gas is absorbed in an amount of 45NL to 85NL per unit amount (kg) at 40°C and 1 atm for 100 minutes from the start of the absorption. The temperature is preferably 45NL or more and 80NL or less, more preferably 50NL or more and 80NL or less, and still more preferably 50NL or more and 75NL or less. When the amount is 65NL or more and 75NL or less, the acid gas absorbent exhibits a higher absorption performance. On the other hand, when the amount is 55NL or more and less than 65NL, the acid gas absorbent exhibits a higher release performance. The method for measuring the amount of released acid gas and the amount of absorbed acid gas will be described later.

In addition, optional components include, for example, an antioxidant, a pH adjusting agent, an antifoaming agent, and an anticorrosive.

The antioxidant can prevent deterioration in acid gas absorbent and improve life thereof. The acid gas absorbent according to the present embodiment has a property of being less likely to be oxidatively degraded as compared with the conventional example. Preferable specific examples of the antioxidant include dibutylhydroxytoluene (BHT), butylhydroxyanisole (BHA), sodium erythorbate, sodium nitrite, sulfur dioxide, 2-mercaptoimidazole, and 2-mercaptobenzimidazole. When the antioxidant is used, the content rate thereof based on the total mass of the acid gas absorbent is preferably 0.01 mass% or more and 1 mass% or less, and particularly preferably 0.1 mass% or more and 0.5 mass%

Preferable specific examples of the antifoaming agent can include a silicone-based antifoaming agent and an organic antifoaming agent. The acid gas absorbent according to the present embodiment has a property of being less likely to foam as compared with the conventional example, and it is possible to reduce the amount of the antifoaming agent mixed. When the antifoaming agent is used, a content rate thereof based on the total mass of the acid gas absorbent is preferably 0.00001 mass% or more and 0.001 mass% or less, and particularly preferably 0.0005 mass% or more and 0.001 mass% or less. The antifoaming agent can prevent foaming of the acid gas absorbent, suppress, for example, a decrease in absorption efficiency and release efficiency of the acid gas, and prevent a decrease in fluidity or circulation efficiency of the acid gas absorbent.

Preferable specific examples of the anticorrosive include phosphoric acid esters, tolyltriazoles, and benzotriazoles. When the anticorrosive is used, the content rate thereof based on the total mass of the acid gas absorbent is preferably 0.00003 mass% or more and 0.0008 mass% or less, and particularly preferably 0.00005 mass% or more and 0.005 mass% or less. Such an anticorrosive can prevent corrosion of plant equipment and improve life thereof.

The acid gas absorbent may be impregnated in a porous support having pores of angstrom size or nanometer size. This makes it possible to further improve the oxidation resistance as compared with the case where the acid gas absorbent is used alone. In addition, since the molecular weight is low, the amount of acid gas absorbed per unit volume can be increased.

The porous support may be any member that can hold the acid gas absorbent and that can structurally withstand the thermally and chemically severe environment when the acid gas is absorbed and released. Specifically, silica, alumina, silica-alumina, titania, zirconia, ceria, carbon, activated carbon, zeolite, zeolite analogous compounds, layered clay compounds, layered double hydroxides, polymethyl methacrylate, metal-organic frameworks (MOF), covalent-organic frameworks (COF), and the like can be used. These porous supports may be used alone or in combination of two or more.

Examples of the structure of the porous support include a honeycomb, a filter, a mesh, a felt, an aerogel, a xerogel, mesoporous silica, and porous particles.

The porous support preferably has a hydrophilic surface. The surface modification method for the porous support includes physical adsorption and chemical bonding, and it is desirable to form chemical bonding in order to ensure stable use. From such a viewpoint, it is desirable that the porous support itself has hydrophilicity or is imparted with hydrophilicity. Examples of the hydrophilic support include a porous support having a functional group capable of forming a hydrogen bond, such as a hydroxyl group, a carboxyl group, a sulfonic acid group, a phosphonic acid group, or an amino group, on the pore surface, and a porous support having an ionic bonding structure on the pore surface, such as zeolite or MOF. In the case of imparting hydrophilicity, for example, silica is mixed with a hydrophilic binder such as polyvinyl acetate or polyvinyl acetal, or hydrophilic fibers, and the mixture is granulated, whereby hydrophilicity can be imparted.

As a method for supporting and modifying the amine compound on the porous support, a generally known method can be applied. Specific examples of the method include bonding by van der Waals force such as impregnation, vacuum impregnation, evaporation to dryness, coating, spray coating, and dip coating, and chemical bonding such as dehydration condensation, dealcoholization reaction, and silane coupling reaction. When the porous support is modified with a solution of an amine compound, the concentration of the amine compound solution is preferably 0.5% or more and 50% or less, the concentration of an amine having a relatively low viscosity is preferably 1% or more and 50% or less, and the concentration of an amine having a high viscosity is preferably 0.5% or more and 10% or less. When the concentration of the amine compound in the aqueous amine solution is equal to or higher than the lower limit value, the amount of the amine compound supported on the porous support can be sufficiently secured, and the amount of the waste liquid after the supporting treatment can be reduced. In addition, when the content is set to be equal to or less than the upper limit value, it is possible to further suppress the adsorption performance of the absorbent from being deteriorated due to the clogging of the pores of the porous particles by the amine compound. That is, by setting the concentration of the amine compound within this range, the amine compound can be modified at an appropriate ratio at the point where the amine compound on the surface of the porous support is modified, and the effect of the amine modification per unit weight / unit volume of the porous support can be enhanced.

Additionally, it is preferable that the porous support has a particle size of the bese material of 0.1mm or more and 5mm or less, a specific surface area of 30m²/g or more and 5000m²/g or less, and a pore volume of 0.05cm³/g or more and 3cm³/g or less, more preferably 0.1cm³/g or more and 1.3cm³/g or less. When the specific surface area and the pore volume are within these ranges, a porous support on which an amine is effectively supported can be obtained. Here, the specific surface area can be evaluated by a method in which molecules having a known adsorption occupied area are adsorbed on the surface of the powder particles, and the specific surface area of the sample is obtained from the amount of the molecules. For example, the mixture is subjected to vacuum deaeration at 100°C for 8 hours using a pretreatment apparatus BELPREP-VAC II manufactured by MicrotracBEL Corp. Thereafter, the specific surface areas are measured at the adsorption temperature 77K using a specific surface area-pore distribution analyzer BELSORP-MINI II manufactured by MicrotracBEL Corp. For example, nitrogen is used as the adsorption gas. When the gas to be adsorbed is CO₂, CO₂ is adsorbed on the CO₂ active sites inside and outside the pores of the support, and therefore, the total amount of CO₂ adsorbed can be evaluated. As the number of reaction active sites increases, the number of opportunities to contact carbon dioxide increases, and thus the adsorption amount increases. Therefore, it is preferable to use a porous support having a specific surface area of about 10 m²/g to several thousand m²/g.

Preferably, the acid gas absorbent according to the embodiment does not contain a low-boiling-point material, specifically, a compound having a boiling point of lower than 100°C. The reason for this is as follows: the acid gas absorbent is heated in a process of removing the acid gas or recovering the acid gas, and thus the low-boiling-point material evaporates and is released into the atmosphere, or the concentration thereof decreases, so that the acid gas removal efficiency changes. Specifically, the content rate of the material having a boiling point of lower than 100°C is preferably 1 mass% or less, and more preferably 0.1 mass% or less, based on the total mass of the acid gas absorbent.

As described above, the acid gas absorbent of the present embodiment is further improved in the amount of absorption and the amount of release of acid gas (particularly, carbon dioxide) per unit mole, the amount of absorption and the amount of release of acid gas per unit volume of the acid gas absorbent, and the acid gas absorption rate and the acid gas release rate, as compared with the conventional examples. Accordingly, the acid gas removal apparatus described below can be miniaturized, that is, made compact, compared to the related art.

In addition, the acid gas absorbent of the present embodiment has high reactivity with acid gases (for example, carbon dioxide CO₂, hydrogen sulfide (H₂S), and carbonyl sulfide (COS)) and excellent solubility in water, and thus is less likely to be precipitated during acid gas absorption and is less likely to be oxidatively degraded. Therefore, the number of times of replacement of the acid gas absorbent when the acid gas removal apparatus is operated can be reduced. Thus, the acid gas removal apparatus can be continuously operated stably for a long time.

### [Method for removing acid gas]

The method for removing an acid gas according to the embodiment of the present invention includes bringing a gas containing an acid gas into contact with a first acid gas absorbent or a second acid gas absorbent, as described above, to remove the acid gas from the gas containing the acid gas.

The method for removing an acid gas according to the embodiment of the present invention has a basic configuration including: a step of absorbing an acid gas into the acid gas absorbent according to the embodiment of the present invention (absorption step); and a step of releasing the acid gas from the acid gas absorbent according to the embodiment of the present invention which has absorbed the acid gas. That is, the basic configuration of the method for removing an acid gas according to the embodiment of the present invention includes: a step of bringing a gas containing an acid gas (for example, an exhaust gas or the like) into contact with the acid gas absorbent to cause the acid gas absorbent to absorb the acid gas (acid gas absorption step); and a step of heating the acid gas absorbent which has absorbed the acid gas, obtained in the acid gas absorption step, to release and remove the acid gas (acid gas separation step).

A method for bringing the gas containing the acid gas into contact with an aqueous solution containing the acid gas absorbent is not particularly limited, and can be performed, for example, by a method in which the gas containing the acid gas is bubbled into the acid gas absorbent to cause the absorbent to absorb the acid gas, a method in which the acid gas absorbent is dropped in a mist form into a gas flow containing the acid gas (atomizing or spraying method), a method in which the gas containing the acid gas and the acid gas absorbent are brought into countercurrent contact with each other in an absorber containing a filler made of porcelain or metal mesh, or the like.

A temperature of the acid gas absorbent when the gas containing the acid gas is absorbed into the aqueous solution is usually preferably from room temperature to 60°C or lower. The temperature is more preferably 50°C or lower, and particularly preferably 20°C or higher and 45°C or lower. The lower the temperature is, the more the amount of the acid gas to be absorbed increases, but a lower limit value of the treatment temperature can be determined by a gas temperature in the process, a heat recovery target, and the like.

A pressure during absorption of the acid gas is usually almost atmospheric pressure. Although it is also possible to increase the pressure to a higher level in order to enhance the absorption performance, it is preferable to absorb the acid gas under atmospheric pressure in order to suppress the energy consumption required for compression.

Examples of a method for separating the acid gas from the acid gas absorbent that has absorbed the acid gas and recovering pure or high-concentration carbon dioxide include a method of heating the acid gas absorbent in the same manner as distillation, foaming the acid gas absorbent in a pot, and releasing the acid gas, and a method of heating the acid gas absorbent while expanding a liquid interface in a shelf tower, a spray tower, or a regeneration tower containing a filler made of porcelain or metal mesh. As a result, the acid gas is liberated and released from carbamate anions and bicarbonate ions.

A temperature of the acid gas absorbent during separation of the acid gas is usually 70°C or higher, preferably 80°C or higher, and more preferably 90°C or higher and 120°C or lower. Although the amount of the acid gas to be released increases as the temperature increases, the energy required for heating the absorbent increases when the temperature increases, and thus the temperature can be determined according to the gas temperature in the process, the heat recovery target, and the like.

A pressure during separation of the acid gas can be usually about 1 to 3 atm. Although it is also possible to reduce the pressure to a lower level in order to enhance the separation performance, it is preferable to separate the acid gas under a pressure within this range in order to suppress the energy consumption required for pressure reduction. The acid gas absorbent after separation of the acid gas can be sent to the acid gas absorption step again for cyclic use (recycle). In addition, heat generated during absorption of the acid gas is generally cooled by heat exchange in a heat exchanger for preheating the aqueous solution to be injected into the regenerator in a process of recycling the aqueous solution.

The purity of the thus recovered acid gas is usually as high as about 95 to 99 vol%. The pure acid gas or the high-concentration acid gas can be used as a synthetic raw material for a chemical product or a polymer substance, a cooling agent for freezing foods, or the like. In addition, it is also possible to isolate and store the recovered acid gas in the underground or the like that is now under technical development.

Among the steps described above, the step of separating the acid gas from the acid gas absorbent and regenerating the acid gas absorbent consumes the largest amount of energy, and, in this step, about 50 to 80% of the energy required for all the steps may be consumed. Therefore, by reducing the energy to be consumed in the acid gas absorbent regeneration step, a cost of the process for absorption and separation of the acid gas can be reduced, and the acid gas can be economically advantageously and efficiently removed from the exhaust gas. According to the present embodiment, energy required for acid gas separation (regeneration step) can be reduced by using the acid gas absorbent of the embodiment described above. Therefore, the process for absorption and separation of carbon dioxide can be efficiently performed under economically advantageous conditions.

In addition, the amine compound according to the embodiment described above has a remarkably high corrosion protection property with respect to metal materials such as carbon steel, as compared with alkanolamines such as 2-aminoethanol which have been conventionally used as an acid gas absorbent. Therefore, the acid gas removal method using such an acid gas absorbent, when adopted, does not require use of high-cost high-grade corrosion resistant steel in plant construction or the like, which is advantageous in terms of cost.

### [Acid gas removal apparatus]

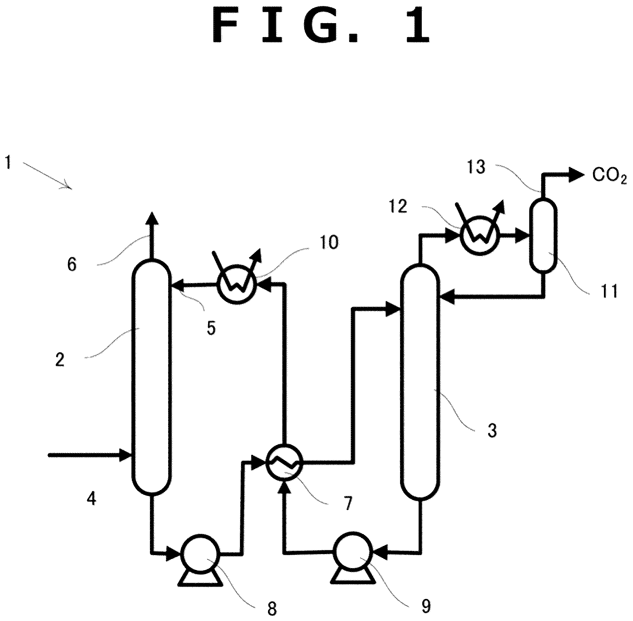

The acid gas removal apparatus according to the embodiment of the present invention includes: an absorber that removes an acid gas from a gas containing the acid gas by causing the first acid gas absorbent or the second acid gas absorbent to absorb the acid gas by contact between the gas containing the acid gas and the acid gas absorbent; and a regenerator that releases the acid gas from the acid gas absorbent that has absorbed the acid gas to regenerate the acid gas absorbent, in which the acid gas absorbent that has been regenerated by the regenerator is reused by the absorber. FIG. 1 is a schematic diagram of an acid gas removal apparatus according to an embodiment.

An acid gas removal apparatus 1 includes: an absorber 2 that brings a gas containing an acid gas (for example, exhaust gas) into contact with an acid gas absorbent and absorbs and removes the acid gas from the gas containing the acid gas; and a regenerator 3 that separates the acid gas from the acid gas absorbent that has absorbed the acid gas and regenerates the acid gas absorbent. Hereinafter, a case where the acid gas is carbon dioxide will be described as an example.

FIG. 1 is a schematic view of an acid gas removal apparatus according to an embodiment.

The acid gas removal apparatus 1 includes: the absorber 2 that brings a gas containing an acid gas (for example, exhaust gas) into contact with an acid gas absorbent and absorbs and removes the acid gas from the gas containing the acid gas; and the regenerator 3 that separates the acid gas from the acid gas absorbent that has absorbed the acid gas and regenerates the acid gas absorbent. Hereinafter, a case where the acid gas is carbon dioxide will be described as an example.

As illustrated in FIG. 1, an exhaust gas containing carbon dioxide such as a combustion exhaust gas discharged from a thermal power plant or the like is guided to a lower portion of the absorber 2 through a gas supply port 4. This exhaust gas is pushed into the absorber 2 and comes into contact with the acid gas absorbent supplied from an acid gas absorbent supply port 5 in an upper portion of the absorber 2. As the acid gas absorbent, the acid gas absorbent according to the embodiment described above is used.

In addition to the amine-based compound and the solvent such as water, the acid gas absorbent may contain other compounds such as a nitrogen-containing compound that improves a carbon dioxide absorption performance, an antioxidant, and a pH adjuster in an arbitrary ratio.

Thus, when the exhaust gas comes into contact with the acid gas absorbent, carbon dioxide in the exhaust gas is absorbed and removed by the acid gas absorbent. The exhaust gas from which carbon dioxide has been removed is discharged from the gas discharge port 6 to the outside of the absorber 2.

The acid gas absorbent that has absorbed carbon dioxide is fed to a heat exchanger 7 by a rich liquid pump 8, and further fed to the regenerator 3. The acid gas absorbent fed into the regenerator 3 moves from an upper portion to a lower portion of the regenerator 3, and, during this time, the acid gas in the acid gas absorbent is released, and the acid gas absorbent is regenerated.

The acid gas absorbent regenerated by the regenerator 3 is fed to the heat exchanger 7 and an absorbent cooler 10 by a lean liquid pump 9, and returned from the acid gas absorbent supply port 5 to the absorber 2.

On the other hand, the acid gas separated from the acid gas absorbent comes into contact with reflux water supplied from a reflux drum 11 in the upper portion of the regenerator 3, and is discharged to the outside of the regenerator 3.

The reflux water in which carbon dioxide is dissolved is cooled by a reflux condenser 12, and then separated from a liquid component in which water vapor accompanied with carbon dioxide is condensed, in the reflux drum 11. This liquid component is guided to the acid gas recovery step by a recovery acid gas line 13. On the other hand, the reflux water from which the acid gas has been separated is fed to the regenerator 3.

According to the acid gas removal apparatus 1 of the present embodiment, it is possible to efficiently absorb and remove the acid gas by using the acid gas absorbent having excellent acid gas absorption characteristics and acid gas release characteristics.

The reflux water from which the acid gas has been separated is fed to the regenerator 3 by the reflux water pump 14. According to the acid gas removal apparatus 1 of the present embodiment, it is possible to efficiently absorb and remove the acid gas by using the acid gas absorbent having excellent acid gas absorption characteristics and acid gas release characteristics.

Hereinafter, embodiments of the present invention will be described in more detail using examples.

### Synthesis of [1-[2-(N-isopropylamino)ethyl]piperazine (1a-1)

To a four-flask equipped with a reflux condenser equipped with an argon inlet tube, a thermometer, a dropping funnel and a mechanical stirrer, 1-(2-aminoethyl) piperidine (0.5mol) and anhydrous acetone (2.0mol) were added under an argon atmosphere, and the mixture was heated under reflux for 24 hours. The reaction mixture was freed from excess acetone and concentrated to give the imine derivative. Absolute ethanol (500mL) was added to the imine-derivative to dissolve it. While the flask was cooled with a water bath, NaBH₄ (0.5mol) was added thereto in small portions. After the addition, the mixture was reacted at room temperature for 2 hours, and then water was added in 50mL to stop the reaction. The precipitate was filtered, the solvent was removed, the residue was redissolved in chloroform, and the precipitate was further filtered. After drying the filtrate with anhydrous magnesium sulfate, it was concentrated and purified by distillation under reduced pressure to obtain colorless and transparent 1-[2-(N-isopropylamino)ethyl]piperazine (1a-1).

### Synthesis of [1-[3-(N-isopropylamino)propyl]piperazine (1a-2)

To a four flask equipped with a reflux condenser equipped with an argon inlet tube, a thermometer, a dropping funnel and a mechanical stirrer, 1-(3-aminoethyl)piperazine (0.5mol) and anhydrous acetone (2.0mol) were added under argon atmosphere, and the mixture was heated under reflux for 24 hours. The reaction mixture was freed from excess acetone and concentrated to give the imine derivative. Absolute ethanol (500mL) was added to the imine-derivative to dissolve it. While the flask was cooled with a water bath, NaBH₄ (0.5mol) was added thereto in small portions. After the addition, the mixture was reacted at room temperature for 2 hours, and then water was added in 50mL to stop the reaction. The precipitate was filtered, the solvent was removed, the residue was redissolved in chloroform, and the precipitate was further filtered. After drying the filtrate with anhydrous magnesium sulfate, it was concentrated and purified by distillation under reduced pressure to obtain colorless and transparent 1-[3-(N-isopropylamino)propyl]piperazine (1a-2).

### [Examples 1 to 6, Comparative Examples 1 to 4]

The following amine compounds were dissolved in water at the concentrations shown in Table 1 to obtain acid gas absorbents.
(1) 1-[2-(N-isopropylamino)ethyl]piperazine (1a-1) 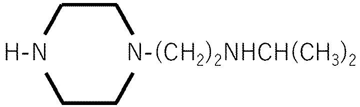
(2) 1-[3-(N-isopropylamino)propyl]piperazine (1a-2) 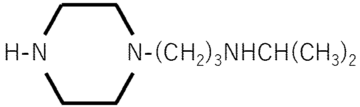
(3) 1-(2-aminoethyl)piperazine (AEPZ) 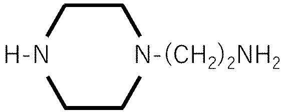
(4) 1-(2-aminopropyl)piperazine (APPZ) 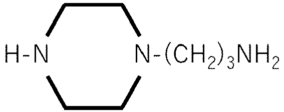
(5) 4-isopropyl-[1-(2-isopolyaminoethyl)]-piperazine (BISMEPEA) 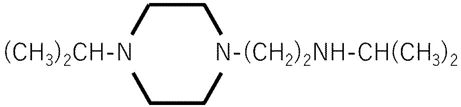
(6) N-[2-(3-hydroxy-1,3-dimethylbutyl)aminoethyl]-1-piperazine (HDMBAEPZ) 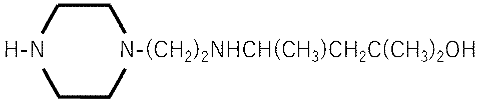
(7) 2-(N-methylamino)ethanol (MEA)

   CH₃NHCH₂CH₂OH
(8) 1-(2-hydroxyethyl)piperazine (HEPZ)

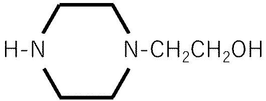

### [Evaluation of amount of carbon dioxide to be absorbed]

Each of these acid gas absorbents was filled in a test tube and heated to 40°C, and a mixed gas containing 10 vol% of carbon dioxide (CO₂) and 90 vol% of a nitrogen (N₂) gas was passed through the acid gas absorbent at a flow rate of 500 mL/min under 1 atm. The concentration of carbon dioxide (CO₂) in the gas at the outlet of the test tube was measured using an infrared gas concentration measuring apparatus (trade name "CGT-700" manufactured by Shimadzu Corporation) to evaluate the absorption performance.

### [Evaluation of amount of carbon dioxide to be released]

The acid gas absorbent that had absorbed carbon dioxide in the above "Evaluation of amount of carbon dioxide to be absorbed" was filled in a test tube and heated to 70°C under 1 atm. The concentration of carbon dioxide (CO₂) in the gas at the outlet of the test tube was measured using an infrared gas concentration measuring apparatus (trade name "CGT-700" manufactured by Shimadzu Corporation), and the release performance was evaluated. The results obtained for the absorption and release performance are shown in Table 1.

### [Accelerated degradation test (oxidation resistance test)]

A degradation test on an acid gas absorbent was performed at a higher temperature than the temperature of 120°C usually assumed in a regeneration tower. In a pressure-resistant sealed container having a volume of 50 ml, 20 ml of each of the acid gas absorbents which had absorbed CO₂ in the above examples and oxygen gas were sealed, and the container was allowed to stand for one week under sealing conditions: at 140°C and in an oxygen atmosphere, and a residual state of amine was examined. The absorbent before and after the degradation test was analyzed using GC/MS, and an amine disappearance weight (k/kg) per kg of the absorbent was calculated, as a disappearance amount, from a decrease in integral value obtained from a chromatogram. The obtained results are as indicated in Table 1.

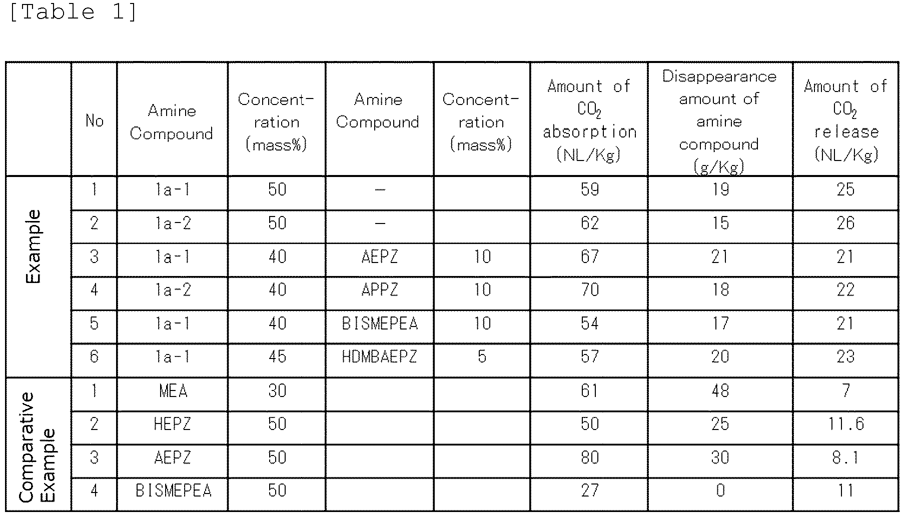

### [Results]

As is clear from the above results, the acid gas absorbent according to the embodiment absorbs a larger amount of carbon dioxide and releases a larger amount of carbon dioxide than the conventional acid gas absorbent. In addition, it is clear that the amine disappearance weight according to the embodiment is very low, and the absorbent is very resistant to deterioration due to thermal cycling in the presence of oxygen.

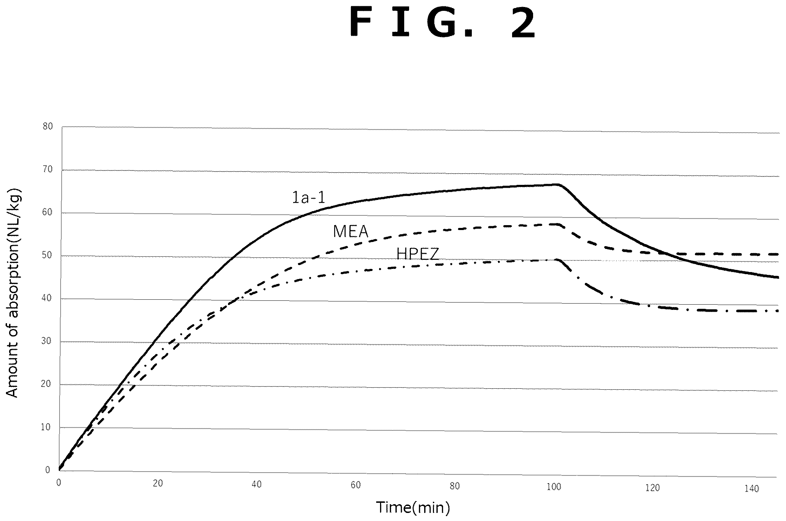

In addition, the acid gas absorbent according to the embodiment has a high absolute amount of carbon dioxide absorption and release and a high carbon dioxide absorption and release rate, compared to the acid gas absorbent in the related art. The results are shown in FIG. 2.

### [Examples 11 to 14, Comparative Examples 11 to 12]

A surface modification method using an amine as an example of the adsorbent will be described. Powdery zeolite (zeolite 4A, zeolite Y-9) available from 500mg was separated as a porous support in a 100mL eggplant flask, and 1-[2-(N-isopropylamino)ethyl]piperazine (1a-1) as an amine was added as a methanol 25mL adjusted to a concentration shown in Table 2, and the mixture was stirred at 25°C for 24 hours. The suspension containing the zeolite was filtered, washed twice with methanol, and dried.

### [Evaluation of specific surface area, pore distribution, and amount of carbon dioxide to be absorbed]

After weighing the glass tube dedicated to the gas adsorption apparatus alone, several tens to several hundreds mg of each of the porous support and the amine-modified product was put into the glass tube dedicated to the gas adsorption apparatus, and vacuum deaeration was performed at 100°C for 8 hours by a pretreatment apparatus BELPREP-VAC II manufactured by MicrotracBEL Corp. Thereafter, the glass tube including the sample was weighed again, and the difference from the glass tube alone was defined as the amount of the sample. After weighing, the adsorption amount at 25°C was measured using a specific surface area / pore distribution analyzer BELSORP-MINI II manufactured by MicrotracBEL Corp., with the adsorbed gas being CO₂.

The adsorption amount was calculated by integrating the adsorption amount of CO₂ when the relative pressure P/P₀ was changed from 10⁻⁶ to 1, where 100kPa (P₀) was the saturated vapor pressure.

The specific surface area was measured in the same manner as in the case of CO₂ except that the non-adsorbed gas was N₂ and the measurement temperature was 77K.

**[TABLE 2]**

| | porous support | Amine Compound | Amine Concentration | S_{BET} (m²/g) | pore volume (cm³/g) | Amount of CO₂ adsorption (mmol/g) |
|---|---|---|---|---|---|---|
| Example11 | Zeolite 4A | 1a-1 | 0.30% | 488 | 0.24 | 3.37 |
| Example12 | Zeolite 4A | 1a-1 | 5.0% | 456 | 0.20 | 3.26 |
| Comparative Example11 | Zeolite 4A | - | - | 537 | 0.25 | 3.11 |
| Example13 | Zeolite Y-9 | 1a-1 | 0.30% | 453 | 0.23 | 3.25 |
| Example14 | Zeolite Y-9 | 1a-1 | 1.0% | 350 | 0.20 | 3.20 |
| Comparative Example12 | Zeolite Y-9 | - | - | 475 | 0.26 | 3.15 |

The evaluation results are shown in Table 2. As is clear from Table 2, the porous support modified with the acid gas absorbent has a larger amount of carbon dioxide absorbed than the conventional porous support.

Embodiments of the present invention include the following configurations.
<1>
   An acid gas absorbent includes
   a solvent, and
   at least one or more amine compounds selected from the group consisting of amine compounds represented by chemical formula (1a). 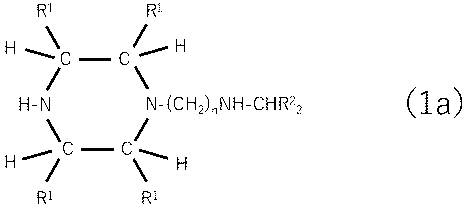 Wherein
      R¹'s are each independently hydrogen or an unsubstituted alkyl group having 3 or less carbon atoms,
      R²'s are each independently an unsubstituted alkyl group having 3 or less carbon atoms,
      n is either 2 or 3.
<2>
   The acid gas absorbent according to <1>, wherein all of the R²'s are unsubstituted alkyl groups having a carbon chain of 1.
<3>
   The acid gas absorbent according to <2>, wherein (CHR²₂) in the chemical formula (1a) is an isopropyl group.
<4>
   The acid gas absorbent according to any one of <1> to <3>, wherein all of the R¹'s are hydrogen.
<5>
   The acid gas absorbent according to any one of <1> to <4>, wherein n is 2.
<6>
   The acid gas absorbent according to any one of <1> to <5>, further including at least one or more amine compounds selected from the group consisting of amine compounds represented by chemical formula (1b), chemical formula (1c), and chemical formula (1d). 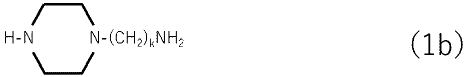 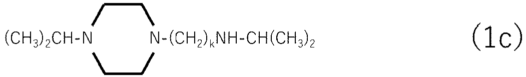 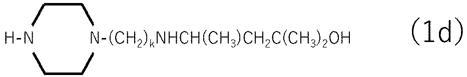 Wherein k is each independently 2 to 3.
<7>
   The acid gas absorbent according to <6>, wherein k is 2.
<8>
   The acid gas absorbent according to <6> or <7>, wherein a content rate of the amine compound represented by chemical formula (1a) is 10 mass% or more and 60 mass% or less, and a content rate of the amine compounds represented by chemical formula (1b) to (1d) is 1 mass% or more and 20 mass% or less, based on the total amount of the acid gas absorbent.
<9>
   The acid gas absorbent according to any one of <1> to <7>, wherein the acid gas absorbent releases acid gas in an amount of 15NL or more and 40NL or less per unit amount (kg) in 40 minutes from the start of release at 70°C and 1 atm, and absorbs acid gas in an amount of 45NL or more and 85NL or less per unit amount (kg) in 100 minutes from the start of absorption at 40°C and 1 atm.
<10>
   The acid gas absorbent according to any one of <1> to <9>, further comprising an antifoaming agent, wherein a content of the antifoaming agent is 0.001 mass% or less with respect to the acid gas absorbent.
<11>
   The acid gas absorbent according to any one of <1> to <10>, wherein the acid gas absorbent is impregnated in a porous support.
<12>
   The acid gas absorbent according to <11>, wherein the porous support includes at least one selected from the group consisting of silica, silica gel, alumina, alumina gel, silica alumina, diatomaceous earth, titania, zirconia, ceria, carbon, activated carbon, zeolite, zeolite analogous compounds, porous glass, vermiculite, layered clay compounds, layered double hydroxides, porous resins, porous fibers, polymethyl methacrylate, MOF, and COF.
<13>
   The acid gas absorbent according to <11> or <12>, wherein the porous support has a hydrophilic surface.
<14>
   The acid gas absorbent according to any one of <11> to <13>, wherein the specific surface area of the porous support is 30m²/g or more and 5000m²/g or less, and the pore volume of the porous support is 0.05cm³/g or more and 3cm³/g or less.
<15>
   A method for removing an acid gas includes bringing a gas containing an acid gas into contact with the acid gas absorbent according to any one of <1> to <14> to remove the acid gas from the gas containing the acid gas.
<16>
   An acid gas removal apparatus includes
   an absorber that removes an acid gas from a gas containing the acid gas by causing the acid gas absorbent according to any one of <1> to <14> to absorb the acid gas by contact between the gas containing the acid gas and the acid gas absorbent, and
   a regenerator that releases the acid gas from the acid gas absorbent that has absorbed the acid gas to regenerate the acid gas absorbent,
   wherein the acid gas absorbent that has been regenerated by the regenerator is reused by the absorber.

While certain embodiments have been described, these embodiments have been presented by way of example only, and are not intended to limit the scope of the inventions. These novel embodiments can be implemented in various other forms, and various combinations, omissions, substitutions, changes, and the like can be made without departing from the spirit of the invention. These embodiments and modifications thereof are included in the scope and spirit of the invention, and are included in the invention described in the claims and the scope of equivalents thereof.

## Claims

1. An acid gas absorbent comprising:
a solvent, and
at least one or more amine compounds selected from the group consisting of amine compounds represented by chemical formula (1a). 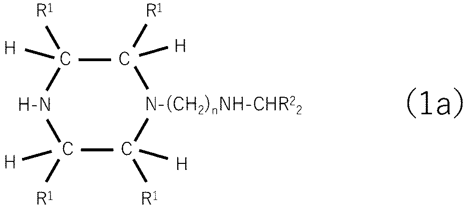
Wherein
R¹'s are each independently hydrogen or an unsubstituted alkyl group having 3 or less carbon atoms,
R²'s are each independently an unsubstituted alkyl group having 3 or less carbon atoms,
n is either 2 or 3.

2. The acid gas absorbent according to claim 1, wherein
all of the R²'s are unsubstituted alkyl groups having a carbon chain of 1.

3. The acid gas absorbent according to claim 2, wherein
(CHR²₂) in the chemical formula (1a) is an isopropyl group.

4. The acid gas absorbent according to claim 1, wherein
all of the R¹'s are hydrogen.

5. The acid gas absorbent according to claim 1, further comprising
at least one amine compound selected from the group consisting of amine compounds represented by chemical formula (1b), chemical formula (1c), and chemical formula (1d). 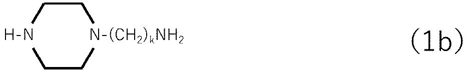 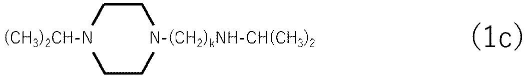 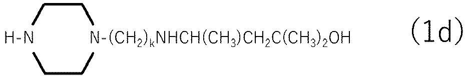
Wherein k is each independently 2 to 3.

6. The acid gas absorbent according to claim 5, wherein
a content rate of the amine compound represented by chemical formula (1a) is 10 mass% or more and 60 mass% or less, and a content rate of the amine compounds represented by chemical formula (1b) to (1d) is 1 mass% or more and 20 mass% or less, based on the total amount of the acid gas absorbent.

7. The acid gas absorbent according to claim 1, wherein
the acid gas absorbent releases acid gas in an amount of 15NL or more and 40NL or less per unit amount (kg) in 40 minutes from the start of release at 70°C and 1 atm, and absorbs acid gas in an amount of 45NL or more and 85NL or less per unit amount (kg) in 100 minutes from the start of absorption at 40°C and 1 atm.

8. The acid gas absorbent according to claim 1, further comprising
an antifoaming agent, wherein
a content of the antifoaming agent is 0.001 mass% or less with respect to the acid gas absorbent.

9. The acid gas absorbent according to claim 1, wherein
the acid gas absorbent is impregnated in a porous support.

10. The acid gas absorbent according to claim 9, wherein
the porous support includes at least one selected from the group consisting of silica, silica gel, alumina, alumina gel, silica alumina, diatomaceous earth, titania, zirconia, ceria, carbon, activated carbon, zeolite, zeolite analogous compounds, porous glass, vermiculite, layered clay compounds, layered double hydroxides, porous resins, porous fibers, polymethyl methacrylate, MOF, and COF.

11. The acid gas absorbent according to claim 9, wherein
the porous support has a hydrophilic surface.

12. The acid gas absorbent according to claim 9, wherein
the specific surface area of the porous support is 30m²/g or more and 5000m²/g or less, and the pore volume of the porous support is 0.05cm³/g or more and 3cm³/g or less.

13. A method for removing an acid gas, comprising:
bringing a gas containing an acid gas into contact with the acid gas absorbent according to claim 1 to remove the acid gas from the gas containing the acid gas.

14. An acid gas removal apparatus (1), comprising:
an absorber (2) that removes an acid gas from a gas containing the acid gas by causing the acid gas absorbent according to claim 1 to absorb the acid gas by contact between the gas containing the acid gas and the acid gas absorbent; and
a regenerator (3) that releases the acid gas from the acid gas absorbent that has absorbed the acid gas to regenerate the acid gas absorbent,
wherein the acid gas absorbent that has been regenerated by the regenerator is reused by the absorber.
